(19) Europäisches Patentamt
European Patent Office
Office européen des brevets

(11) **EP 3 155 597 B1**

(12) **EUROPÄISCHE PATENTSCHRIFT**

(45) Veröffentlichungstag und Bekanntmachung des Hinweises auf die Patenterteilung:
**11.03.2020  Patentblatt 2020/11**

(21) Anmeldenummer: **15753340.7**

(22) Anmeldetag: **13.08.2015**

(51) Int Cl.:
**G06T 19/00** *(2011.01)*

(86) Internationale Anmeldenummer:
**PCT/EP2015/068636**

(87) Internationale Veröffentlichungsnummer:
**WO 2016/026759 (25.02.2016 Gazette 2016/08)**

(54) **REFORMATIERUNG UNTER BERÜCKSICHTIGUNG DER ANATOMIE EINES ZU UNTERSUCHENDEN OBJEKTS**

REFORMATTING WHILE TAKING THE ANATOMY OF AN OBJECT TO BE EXAMINED INTO CONSIDERATION

REFORMATAGE AVEC PRISE EN COMPTE DE L'ANATOMIE D'UN OBJET À ANALYSER

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.08.2014  DE 102014216702**

(43) Veröffentlichungstag der Anmeldung:
**19.04.2017  Patentblatt 2017/16**

(73) Patentinhaber: **Siemens Healthcare GmbH 91052 Erlangen (DE)**

(72) Erfinder:
- **KRETSCHMER, Jan 90429 Nürnberg (DE)**
- **SOZA, Grzegorz 90562 Heroldsberg (DE)**
- **SÜHLING, Michael 91052 Erlangen (DE)**
- **TIETJEN, Christian 90763 Fürth (DE)**

(56) Entgegenhaltungen:
**US-A1- 2008 049 991**

- **KANITSAR A ET AL: "Advanced curved planar reformation: flattening of vascular structures", VIS 2003. IEEE VISUALIZATION 2003. PROCEEDINGS. SEATTLE, WA, OCT. 19 - 24, 2003; [ANNUAL IEEE CONFERENCE ON VISUALIZATION], NEW YORK, NY : IEEE, US, 1. Januar 2003 (2003-01-01), Seiten 43-50, XP031173479, DOI: 10.1109/VISUAL.2003.1250353 ISBN: 978-0-7803-8120-9**
- **AUZINGER THOMAS ET AL: "Vessel Visualization using Curved Surface Reformation", IEEE TRANSACTIONS ON VISUALIZATION AND COMPUTER GRAPHICS, IEEE SERVICE CENTER, LOS ALAMITOS, CA, US, Bd. 19, Nr. 12, 1. Dezember 2013 (2013-12-01), Seiten 2858-2867, XP011529779, ISSN: 1077-2626, DOI: 10.1109/TVCG.2013.215 [gefunden am 2013-10-16]**

EP 3 155 597 B1

## Beschreibung

Die Erfindung betrifft ein Verfahren zum Abbilden eines zu untersuchenden dreidimensionalen Objekts. Zudem betrifft die Erfindung eine Vorrichtung zum Abbilden eines zu untersuchenden dreidimensionalen Objekts.

Computergestützte Visualisierungsverfahren spielen eine wichtige Rolle in der klinischen Anwendung, da sie eine sehr flexible und wirksame Untersuchungsmöglichkeit von aus medizinischen bildgebenden Verfahren gewonnenen Daten zur Verfügung stellen. Die Bedeutung und der positive Einfluss von computergestützten Arbeitsabläufen auf die Gesamteffektivität der heutigen radiologischen Praxis liegen auf der Hand. Allerdings ist es wahrscheinlich, dass in Zukunft die Auswertung der immer größer werden Menge an Bildinformationen von den dafür ausgebildeten Experten nicht mehr ohne zusätzliche Hilfe bewältigt werden kann. Daher ist es entscheidend, die Verarbeitung von medizinischen Bildinformationen effektiver zu gestalten. Das heißt, dass Anwendungen beschleunigt werden müssen, ohne dass dabei die Qualität der Arbeit, insbesondere die Genauigkeit und Vollständigkeit bei der Untersuchung und Auswertung der Bildinformationen verloren geht.

Nicht nur auf dem Gebiet der Onkologie und der Traumatologie sind Rekonstruktionen auf Basis der Computertomographie meist die Basis sowohl für eine Diagnose als auch für die anschließende Behandlung. Bis heute ist das Standardverfahren in der diagnostischen Radiologie eine schichtweise Untersuchung von standardisierten orthogonal zueinander orientierten Ansichten. Leider ist die direkte Betrachtung und Auswertung für viele Aufgaben nicht ideal, da die anatomischen Strukturen im Allgemeinen nicht mit dem Koordinatensystem des Computertomographen bzw. der bildgebenden medizinischen Einrichtung konform verlaufen und üblicherweise komplizierte Formen aufweisen. Beispielsweise treten Knochenmetastasen in Rippen und im Becken bei fortgeschrittenen Krebserkrankungen, insbesondere bei Prostatakrebs oder Brustkrebs, häufig auf, und ihre Entdeckung und das Nachverfolgen der Metastasen stellt sogar für sehr erfahrene Radiologen eine aufwändige und zeitraubende Aufgabe dar.

Aufgrund der genannten Schwierigkeiten wurden Verfahren zur Visualisierung von aufgenommenen medizinischen Bilddaten entwickelt, welche die speziellen anatomischen Gegebenheiten berücksichtigen. Allerdings ist das Erzeugen von aussagekräftigen und für die Diagnose relevanten Visualisierungen von medizinischen Datensätzen eine sehr schwierige Aufgabe. Infolge der hohen geometrischen Komplexität des menschlichen Körpers treten häufig Probleme, wie zum Beispiel wechselseitige Verdeckungen und Störungen der Ansichten, auf. Da die Variation der anatomischen Geometrie zwischen den einzelnen Patienten relativ gering ist, wurden zahlreiche Visualisierungsalgorithmen entwickelt, von denen viele sich auf eine spezielle Form einer bestimmten anatomischen Struktur stützen und beschränken. Bei dieser Gruppe von Verfahren besteht allgemein eine Herangehensweise darin, Organe oder andere anatomische Strukturen durch geometrische Primitive wie Kugeln, Zylinder oder Ebenen anzunähern, was eine einfache Projektion des umgebenden Gewebes ermöglicht. Die genannten Verfahren sind üblicherweise für spezielle Anwendung optimiert und konzentrieren sich auf bestimmte Arten von CTFunktionen. Typische Anwendungen bilden Projektionen von Bereichen des Herzens oder von Tumoren. Obgleich ihre Stärke die Einfachheit und Intuition des Ansatzes bilden, ist es bei Primitiven mit geschlossener Form oft nicht möglich, die Geometrie einer projizierten anatomischen Struktur ausreichend genau nachzubilden, was bei der Darstellung zu Verzerrungen und Verdeckungen führt.

Um eine flexiblere Betrachtung von medizinischen Datensätzen zu ermöglichen, wurde die multiplanare Reformatierung (MPR) entwickelt, um rekonstruierte CTVolumen in beliebig orientierten Ebenen darzustellen.

Eine andere Art der Reformatierung, die kurvenförmige planare Reformatierung (CPR) und ihre Derivate, ermöglichen sogar noch flexiblere Schnitte durch die Datensätze, die durch einzelne geometrische Zentrallinien oder komplexe Zentralliniengraphen definiert werden. CPRs werden üblicherweise bei der Visualisierung von Gefäßen angewandt, da die erzeugten Schnitte eine sorgfältige Untersuchung der Lichtung der Gefäße erlauben und wertvollen anatomischen Kontext umfassen. Im Gegensatz zu MPRs sind CPRs unmittelbar durch patientenspezifische anatomische Daten gesteuert, was letztlich dazu führt, dass mehr Information in einer einzigen, um die Längsachse drehbaren 2DAnsicht, kondensiert wird.

Ein Hauptproblem dieser Art von Abbildungsalgorithmen besteht darin, dass sie sich auf eine bestimmte Art von Projektion stützen. Ferner liefern sie im Allgemeinen keine Werkzeuge zum Bearbeiten von auftretenden Verzerrungen und parametrisieren nur einzelne Schnitte anstatt ganze Volumenbereiche.

Es wurden auch bestimmte Benutzeroberflächen für Visualisierungen von verschiedenen anatomischen Strukturen entwickelt. Verfahren zur zweidimensionalen Darstellung des Dickdarms sind ein Beispiel für Maßnahmen, um die leider sehr fehleranfälligen Diagnoseverfahren von modernen Krebsvorsorgeuntersuchungen zu verbessern. Aufgrund ihrer komplizierten Form und Abmessungen sind Gedärme nur sehr schwer zu visualisieren. Um die spezielle Form von wichtigen Merkmalen wie Polypen auf ausdrucksstarke Weise beizubehalten, sind Darmentfaltungsverfahren im Allgemeinen besonders auf Aspekte der Parametrisierung und der Verdeckung von Merkmalen gerichtet.

Eine im Allgemeinen sehr zeitaufwändige Angelegenheit ist das Aufspüren von Knochenläsionen. Bei komplexen anatomischen Strukturen wie dem Brustkorb steht nicht nur das Aufspüren von Anomalien im Vordergrund. Die entdeckten Metastasen müssen auch dokumentiert werden. Genauer gesagt, müssen die Metastasen zurück zu dem entsprechenden Wirbel verfolgt werden, um sie richtig kennzeichnen zu können. Navigiert man schichtweise durch den Brustkorb, wird dies zu einer mühsamen Aufgabe, da die Querschnitte von Rippenknochen zwischen den Schichten einer Verschiebung unterliegen. Bei modernen Segmentierungsverfahren und Visualisierungsverfahren wurde versucht, dieses Problem zu lösen, indem normalisierte Ansichten erzeugt wurden.

Um Entfaltungen für die Untersuchung des Schädels bereitzustellen, wurden Screeningverfahren zur Detektion von Traumata entwickelt. Mit dem speziell für den Schädel entwickelten Algorithmus wird ein elastisches Gitter über den Kopf des Patienten gelegt, um den Schädelknochen einzuhüllen bzw. zu parametrisieren. Anschließend wird das Gitter genutzt, um Projektionen zu berechnen, die im Vergleich zu den Schnittansichten eine bessere Übersichtlichkeit und eine erleichterte Verfolgbarkeit von Schädelverletzungen ermöglichen.

Bei den kurz skizzierten Reformatierungsverfahren treten unterschiedliche Schwierigkeiten auf, die im Folgenden kurz erläutert werden sollen. Ein Problem besteht darin, dass bei einer normalisierten Darstellung oder einer Flächendarstellung einer ursprünglich sehr viel ausgeprägteren Struktur unweigerlich Verzerrungen auftreten. Diese Verzerrungen treten sowohl auf dem Gebiet der Abbildung von Mannigfaltigkeiten als auch auf dem Gebiet der Darstellung von Volumen auf. Das Vorgehen bei ersterem sei im Folgenden als Oberflächenparametrisierung und bei letzterem als Volumenparametrisierung bezeichnet. Das Problem der Verzerrungen und der Minimierung derselbigen bei der Darstellung von Objekten mit verschiedenen Formen tritt häufig bei der Bearbeitung von Computergraphiken auf. Beispielsweise tritt es bei TexturAbbildungen, Krümmen von Umgebungsbereichen und der Deformationen von oberflächenbasierten Netzen und Volumennetzen auf.

Konforme Abbildungen haben die Eigenschaft, winkelgetreu abzubilden, was besonders wichtig ist, wenn man die Ähnlichkeit des abzubildenden Gegenstands bei der Projektion beibehalten will. Die Konformität der Abbildungen spielt eine wichtige Rolle bei den medizinischen Bildregistrierungen. Allerdings kommt es auch bei konformen Abbildungen zu lokalen oder globalen Skalierungen. Dies führt zu einer vergrößerten oder verkleinerten Darstellung einzelner Bereiche und ergibt ein unnatürlich aussehendes Parametrisierungsergebnis. Es gibt auch Ansätze, bei denen versucht wird, das Ziel der Konformität und der Längentreue (im Englischen rigidity) miteinander zu vereinbaren, was auch als ARAPParadigma (ARAP = as rigid as possible) bezeichnet wird. Dabei werden insbesondere auf lokaler Ebene übermäßige Längenverzerrungen bei größtmöglicher Beibehaltung der Winkeltreue vermieden. Dadurch wird insbesondere verhindert, dass einige Bereiche viel zu groß oder zu klein abgebildet werden und so die Übersichtlichkeit und Benutzerfreundlichkeit der Darstellung gewährleistet ist.

ARAP wurde bisher verwendet, um Dreiecksgitter zu deformieren (siehe Sorkine et al. "As rigid as possible surface modeling", Symposium on Geometriy processing, volume 4, 2007), um Oberflächen zu parametrisieren (siehe Liu et al. "A local/global approach to mesh parameterization", Computer Graphics Forum, volume 27, pages 14951504, Wiley Online Library, 2008) und um Volumen zu deformieren (siehe Zollhöfer et al. "GPU based ARAP deformation using volumetric lattices", Eurographics 2012Short Papers, pages 8588, The Eurographics Association, 2012). Weiterer relevanter Stand der Technik ist aus US2008049991 A1, "SYSTEM AND METHOD FOR FLATTENED ANATOMY FOR INTERACTIVE SEGMENTATION AND MEASUREMENT", und Kanitsar A et al., "Advanced curved planar reformation: flattening of vascular structures" (IEEE VISUALIZATION 2003), bekannt.

Allerdings bestehen bei den herkömmlichen Ansätzen der zweidimensionalen Darstellung zu untersuchender Objekte oder Bereiche immer noch Einschränkungen bezüglich der allgemeinen Anwendbarkeit der Verfahren, da sie üblicherweise auf die Darstellung bestimmter Organe oder bestimmter anatomischer Einheiten, wie zum Beispiel Schädelknochen, beschränkt sind. Oft ist es jedoch notwendig, zunächst breitere Bereiche abzubilden, die anschließend auf Anomalien untersucht werden sollen. Somit besteht ein Nachteil der herkömmlichen Ansätze darin, dass sie nur auf bestimmte anatomische Strukturen anwendbar sind und darüber hinaus auch nicht einfach auf diese umgebende Bereiche ausdehnbar sind, was zu einer geringen Flexibilität der herkömmlichen Verfahren und zu einem beschränkten Anwendungsgebiet dieser Verfahren führt. Weiterhin besteht auch das Problem der verzerrten zweidimensionalen Abbildungen. Zwar gibt es Verfahren, die, wie kurz beschrieben, Verzerrungen minimieren können, allerdings sind die Verzerrungen grundsätzlich nicht zu eliminieren, da immer ein Kompromiss zwischen Winkeltreue und Längentreue eingegangen werden muss.

Es kann daher eine Aufgabe darin gesehen werden, ein breit anwendbares Verfahren zu entwickeln, mit dem eine möglichst wirklichkeitsgetreue Übersichtsdarstellung eines dreidimensionalen zu untersuchenden Bereichs möglich ist. In der gesuchten Übersichtsdarstellung soll nicht nur eine Schicht, sondern auch die Umgebung, also das Objekt in seiner vollen Breite und Dicke darstellbar sein.

Diese Aufgabe wird durch ein Verfahren gemäß Patentanspruch 1 und durch eine Vorrichtung gemäß Patentanspruch 14 gelöst.

Bei dem erfindungsgemäßen Verfahren wird zunächst eine dreidimensionale parametrisierte Fläche festgelegt, welche konform zu der anatomischen Struktur des zu untersuchenden dreidimensionalen Objekts ist. Die dreidimensionale parametrisierte Fläche wird anschließend auf eine zweidimensionale parametrisierte Fläche abgebildet. Schließlich wird das zu untersuchende dreidimensionale Objekt durch Abbilden von der dreidimensionalen parametrisierten Fläche zugeordneten Bildpunkten auf die zweidimensionale parametrisierte Fläche zweidimensional dargestellt. Konform soll in diesem Zusammenhang bedeuten, dass eine dreidimensionale Fläche an die Gestalt eines dreidimensionalen Objekts angepasst wird. Dies kann beispielsweise durch ein Näherungsverfahren, zum Beispiel durch ein Verfahren, welches auf der Methode der kleinsten Quadrate oder ähnlichen Regressionsverfahren oder anderen Optimierungsansätzen beruht, oder auch durch ein intuitives Anpassen durch einen Benutzer erreicht werden. Konform soll in diesem Zusammenhang also bedeuten, dass die parametrisierte Fläche die Mitte des Objekts beschreibt oder dass sie die Oberfläche des Objekts beschreibt oder dass sie eine andere Fläche von Wichtigkeit durch das Objekt bzw. ein Organ bzw. die Struktur beschreibt.

Bei der Ermittlung und Optimierung der zweidimensionalen parametrisierten Fläche können ARAPOptimierungsverfahren zur Anwendung kommen. Im Gegensatz zu herkömmlichen Verfahren dient jedoch als Ausgangsfläche eine genau an die Anatomie angepasste Fläche.

Eine der Erfindung zugrundeliegende Idee kann darin gesehen werden, dass die Reformatierung der zu untersuchenden Bereiche durch die Anatomie von auch sehr unterschiedlichen zu untersuchenden Objekten in einem breiten Bereich bestimmt wird. Beispielsweise können mit dem erfindungsgemäßen Verfahren komplexe und ausgedehnte Strukturen wie zum Beispiel Knochenskelette erfasst und so dargestellt werden, dass sie schnell untersucht werden können. Die erwähnte dreidimensionale parametrisierte Fläche kann sowohl kontinuierlich als auch diskret parametrisiert sein.

Die erfindungsgemäße Vorrichtung zum Abbilden eines zu untersuchenden dreidimensionalen Objekts umfasst eine ParameterflächenErmittlungseinheit. Die ParameterflächenErmittlungseinheit ist dazu eingerichtet, eine dreidimensionale parametrisierte Fläche festzulegen, welche konform zu der anatomischen Struktur des zu untersuchenden dreidimensionalen Objekts ist. Weiterhin umfasst die erfindungsgemäße Vorrichtung eine Reformatierungseinheit, welche dazu eingerichtet ist, die dreidimensionale parametrisierte Fläche auf eine zweidimensionale parametrisierte Fläche abzubilden. Zudem weist die erfindungsgemäße Vorrichtung eine Abtasteinheit auf, welche dazu eingerichtet ist, das zu untersuchende dreidimensionale Objekt durch Abbilden von der dreidimensionalen parametrisierten Fläche zugeordneten Bildpunkten auf die zweidimensionale parametrisierte Fläche abzubilden. Unter einem dreidimensionalen Objekt können sowohl ein ganzes Organ oder Körperteil als auch Abschnitte davon bzw. irgendein Teilvolumen eines dreidimensionalen Objekts verstanden werden.

Bei dem Verfahren zur Bestimmung einer Kameraposition in einer dreidimensionalen Bildaufnahme eines zu untersuchenden Objekts wird die Kameraposition orthogonal zu einem Feld von UpVektoren festgelegt. Die UpVektoren werden mit einer VertexSkinningTechnik, angewandt auf eine das zu untersuchende Objekt definierende dreidimensionale parametrisierte Fläche, ermittelt. Dabei kann die genannte dreidimensionale parametrisierte Fläche die bereits definierte dreidimensionale parametrisierte Fläche sein. Sie kann aber auch eine Einhüllende dieser Fläche sein, die eine "sanftere" Geometrie mit weniger starken Krümmungen als die genannte dreidimensionale parametrisierte Fläche aufweist. Die UpVektoren zeigen in Aufwärtsrichtung eines Sichtbereichs oder eines Bildes.

Bei dem Verfahren zur Darstellung eines Teilbereichs eines zu untersuchenden Objekts wird zunächst das erfindungsgemäße Verfahren zum zweidimensionalen Abbilden eines zu untersuchenden dreidimensionalen Objekts ausgeführt. Anschließend wird ein darzustellender Teilbereich des zu untersuchenden Objekts durch Kennzeichnen in der zweidimensionalen Darstellung festgelegt. Darauf wird ein geeignetes Visualisierungsverfahren in dem dazustellenden Teilbereich ausgeführt. Beispielsweise kann es vorkommen, dass bei der Übersichtsdarstellung des zu untersuchenden Objekts ein Teilbereich nicht optimal dargestellt ist. Dieser Teilbereich kann anschließend zur eingehenderen Begutachtung durch ein spezielles Visualisierungsverfahren vorbereitet werden. Dazu kann in dem Übersichtsbild zum Beispiel der Verlauf einer Linie durch einen Bereich eingezeichnet werden, wobei der von der Linie gekennzeichnete Bereich vollständig durch das zusätzliche Visualisierungsverfahren erfasst werden soll. Die Linie kann zum Beispiel ein sogenannter Spline sein.

Die Erfindung umfasst auch ein Computerprogrammprodukt, welches direkt in einen Prozessor einer programmierbaren Auswertungseinrichtung einer medizintechnischen bildgebenden Anlage ladbar ist, mit ProgrammcodeMitteln, um alle Schritte der erfindungsgemäßen Verfahren auszuführen, wenn das Programm in der Auswertungseinrichtung ausgeführt wird.

Eine solche softwaremäßige Realisierung hat den Vorteil, dass bisherige Auswertungseinrichtungen von medizinischen bildgebenden Einrichtungen durch Implementierung des Programms in geeigneter Weise modifiziert werden können, um in der erfindungsgemäßen Weise eine Auswertung von erzeugten Bilddaten durchzuführen, was z.B. mit den oben genannten Vorteilen verbunden ist.

Weitere, besonders vorteilhafte Ausgestaltungen und Weiterbildungen der Erfindung ergeben sich aus den abhängigen Ansprüchen sowie der nachfolgenden Beschreibung, wobei die unabhängigen Ansprüche einer Anspruchskategorie auch analog zu den abhängigen Ansprüchen einer anderen Anspruchskategorie weitergebildet sein können.

In einer bevorzugten Ausgestaltung des Verfahrens zum Abbilden eines zu untersuchenden dreidimensionalen Objekts wird die dreidimensionale parametrisierte Fläche durch ein dreidimensionales Flächennetz mit einer Vielzahl von Einzelelementen parametrisiert. In dieser Ausgestaltung wird also eine diskrete Parametrisierung der dreidimensionalen parametrisierten Fläche gewählt. Diese diskrete Parametrisierung lässt sich mit relativ geringem Datenverarbeitungsaufwand und mit hoher Flexibilität realisieren. Mit dem gewählten Flächennetz wird die zweidimensionale parametrisierte Fläche durch ein zweidimensionales Netz mit einer Vielzahl von Einzelelementen parametrisiert. Das zweidimensionale Darstellen des zu untersuchenden dreidimensionalen Objekts erfolgt durch Abbilden von den Einzelelementen des dreidimensionalen Flächennetzes zugeordneten Bildpunkten auf die Einzelelemente des zweidimensionalen Netzes. Dieser Vorgang wird im Englischen auch als Resampling bezeichnet.

Beispielsweise lässt sich der letzte Schritt so realisieren, dass den einzelnen Pixeln des zweidimensionalen Netzes die zugehörigen Bildpunkte des dreidimensionalen Flächennetzes in der dreidimensionalen Darstellung zugeordnet werden. Dafür werden die Pixel des zweidimensionalen Netzes zunächst durch die Einzelelemente, in denen sie liegen, parametrisiert, diese Parametrisierung auf die Einzelelemente des dreidimensionalen Flächennetzes übertragen und der zugehörige Bildpunkt in der dreidimensionalen Darstellung ermittelt.

Wie bereits erwähnt, besteht ein Schritt zur Erfassung eines abzubildenden Objekts bzw. der dazugehörenden Geometrie in der Parametrisierung, beispielsweise durch Segmentierung, dieses Objekts. Dabei können zum Beispiel modellbasierte parametrisierte Bildsegmentierungen verwendet werden, die auf statistischen Datenbanken beruhen. Zur Anpassung der Segmentierung an das konkrete Objekt werden zunächst zur Initialisierung für die einzelnen Parameter statistische Mittelwerte genutzt. Die Parameter werden anschließend optimiert, bis eine bestmögliche Anpassung eines aus Segmenten bestehenden Netzes und den Abmessungen des Objekts erreicht ist. Eine Anpassung eines modellbasierten Netzes kann beispielsweise auch durch die medizinische Bildregistrierung ermöglicht werden, wobei Deformationsfelder ermittelt werden, die das Anpassen eines Referenzdatensatzes an einen einem parametrisierten Objekt entsprechenden Zieldatensatz ermöglichen. Unter Verwendung des Deformationsfeldes können beispielsweise bestimmte Strukturen in dem Referenzdatensatz, wie zum Beispiel eine Zentralfläche zwischen zwei Oberflächen von dem Referenzdatensatz auf den patientenspezifischen Datensatz abgebildet werden.

Die dreidimensionale parametrisierte Fläche kann eine offene Fläche sein. Sie kann aber auch eine geschlossene Fläche sein, die vor der Abbildung der dreidimensionalen parametrisierten Fläche auf eine zweidimensionale parametrisierte Fläche in eine offene dreidimensionale parametrisierte Fläche gewandelt wird. Entscheidend ist, dass die dreidimensionale parametrisierte Fläche bei der Reformatierung einen Rand aufweist, welcher auf den Rand eines initialen zweidimensionalen Netzes abgebildet werden kann.

Bei dem Abbilden von den Einzelelementen des dreidimensionalen Flächennetzes zugeordneten Bildpunkten auf die Einzelelemente des zweidimensionalen Netzes kann jeder Pixel in dem zweidimensionalen Netz in Abhängigkeit von seiner Position in einem Einzelelement des zweidimensionalen Netzes parametrisiert werden. Diese Parametrisierung kann anschließend auf das dreidimensionale Flächennetz übertragen werden und es kann für jeden Pixel des zweidimensionalen Netzes eine 3DPosition in dem dreidimensionalen Flächennetz berechnet werden, welche der Position des Pixels in dem zweidimensionalen Netz entspricht. Schließlich kann ein der 3DPosition zugeordneter Bildwert auf den zugehörigen Pixel für jeden Pixel des zweidimensionalen Netzes übertragen werden.

Die Einzelelemente können zum Beispiel Dreiecke und/oder Vierecke und/oder Sechsecke und/oder andere Vielecke umfassen. Das dreidimensionale Flächennetz kann ein reguläres Netz oder ein irreguläres Netz sein. Dabei besteht ein reguläres Netz aus Knoten mit immer den gleichen Valenzen, während dies bei einem irregulären Netz nicht der Fall sein braucht.

Um eine möglichst verzerrungsfreie bzw. verzerrungsarme und sowohl längengetreue als auch winkelgetreue zweidimensionale Abbildung zu erhalten, wird der Schritt des Abbildens der dreidimensionalen parametrisierten Fläche auf die zweidimensionale parametrisierte Fläche durch Optimieren eines der dreidimensionalen parametrisierten Fläche und einer zu optimierenden zweidimensionalen parametrisierten Fläche zugeordneten Energieterms realisiert.

Soll auch das Volumen um eine darzustellende dreidimensionale Struktur bei dem zweidimensionalen Abbilden eines zu untersuchenden dreidimensionalen Objekts berücksichtigt werden, so wird zusätzlich zu der dreidimensionalen parametrisierten Fläche eine Mehrzahl von dreidimensionalen parametrisierten OffsetFlächen, welche konform zu der anatomischen Struktur des zu untersuchenden dreidimensionalen Objekts sind, erzeugt. Es entsteht, anschaulich gesagt, eine Art Stapel aus Flächennetzen bzw. der dazu gehörigen Schichten. Eine Berücksichtigung des Volumens um eine dreidimensionale Flächenstruktur herum ermöglicht nicht nur die Darstellung einer Schicht des zu untersuchenden Objekts, sondern idealerweise die Darstellung des Objekts in seiner vollen Breite und Dicke. Es wird also nicht nur eine Fläche abgebildet, sondern eine ganze Umgebung, also ein Volumen.

Man erhält also eine Parametrisierung der Umgebung, die dann (basierend auf dem Gitter inklusive OffsetFlächen) volumetrisch in eine aufgefaltete Form deformiert wird.

Es werden also quasi Volumen bzw. mehrere Schichten bzw. Schichtstapel aufgefaltet.

Eine Möglichkeit, diese parametrisierten OffsetFlächen zu erzeugen, besteht darin, die dreidimensionalen parametrisierten OffsetFlächen durch Ermitteln von zu der dreidimensionalen parametrisierten Fläche orthogonalen Normalenvektoren festzulegen. Dabei sind die Normalenvektoren auf einen bestimmten Abstand normiert, welcher die Distanz zwischen den OffsetFlächen und der dreidimensionalen parametrisierten Mittenfläche definiert.

Die OffsetFlächennetze der dreidimensionalen parametrisierten OffsetFlächen können durch Anwendung von GitternetzGlättungsverfahren, zum Beispiel LaplaceGitternetzGlättungsverfahren geglättet werden, wobei Überschneidungen von benachbarten Normalenvektoren vermieden werden.

In einer vereinfachten Ausgestaltung des erfindungsgemäßen Verfahrens wird bei dem iterativen Optimierungsverfahren des zweidimensionalen Netzes nur die mittlere parametrisierte Fläche optimiert und es werden die Strukturen der Offsetflächen entsprechend angepasst.

Alternativ können die dreidimensionalen OffsetFlächennetze explizit berechnet werden. Bei der Abbildung dieser dreidimensionalen Flächennetze auf zweidimensionale Flächennetze wird ein den einzelnen Flächennetzen zugeordneter Energieterm mit einem zusätzlichen Scherterm berechnet und iterativ optimiert. Durch Festlegen eines Koeffizienten des Scherterms kann Einfluss darauf genommen werden, wie das Verhältnis der Längentreue zu der Winkeltreue der Abbildung ausgeprägt ist.

Vorteilhaft wird bei der Optimierung des Stapels von Flächennetzen mit dem Energieterm die Längentreue innerhalb der Schichten und die Winkelverzerrung zwischen den Schichten separat modelliert. D.h., die dabei eingesetzten Iterationsverfahren bezüglich der Längentreue und der Winkeltreue lassen sich getrennt ausführen. Der Energieterm erlaubt im Speziellen, die Scherung bzw. die Verzerrung der Schichten gezielt gegeneinander abzuwägen.

Besonders vorteilhaft kann bei dem Abbilden der dreidimensionalen parametrisierten Flächen auf eine zweidimensionale parametrisierte Fläche die lokale Verzerrung der Abbildung in Abhängigkeit von der Wichtigkeit der Bildbereiche des dreidimensionalen Objekts gewählt werden. Bei dieser Variante kann die unterschiedliche Wichtigkeit der Bildbereiche durch Gewichtungsfaktoren in dem zu optimierenden Energieterm berücksichtigt werden. Somit können die besonders wichtigen bzw. interessanten Bildbereiche von Verzerrungen weitgehend frei gehalten werden und die Verzerrungen in weniger wichtige Bildbereiche "verschoben" werden.

Als Anwendung kann zum Beispiel eine Annotation von Punkten und/oder Bereichen und/oder Strukturen vorgenommen werden. Beispielsweise können in der Abbildung Marker gesetzt werden oder Läsionen annotiert werden. Weiterhin kann auch eine interaktive Verfeinerung oder Verschiebung der Abbildung bzw. Reformation basierend auf der aktuellen Lösung durchgeführt werden. Bei der Verschiebung kann die konforme parametrisierte dreidimensionale Fläche in Normalenrichtung oder negativer Normalenrichtung verschoben werden und neu abgetastet werden. Schließlich können auch zusätzlich Detailansichten erzeugt werden. Weil die Visualisierung mit der abzubildenden Struktur konform ist, lassen sich leicht Teilbereiche identifizieren und erneut auffalten.

Bei dem Verfahren zur Darstellung eines Teilbereichs eines zu untersuchenden Objekts kann das Festlegen des darzustellenden Teilbereichs das Einzeichnen einer SplineKurve in dem darzustellenden Teilbereich als Zentrum für eine Bilddarstellung mit einem CPRVerfahren als Visualisierungsverfahren umfassen. Alternativ oder zusätzlich kann als Visualisierungsverfahren auch ein MPRVerfahren verwendet werden.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen oder entsprechenden Bezugsziffern versehen. Die Figuren sind in der Regel nicht maßstäblich. Es zeigen:

Figur 1 ein Flussdiagramm, welches ein Verfahren gemäß einem Ausführungsbeispiel der Erfindung veranschaulicht,

Figur 2 eine schematische Darstellung einer Reformatierung gemäß einem Ausführungsbeispiel der Erfindung,

Figur 3 eine schematische Ansicht eines Abtastschritts zur Erzeugung einer zweidimensionalen Darstellung,

Figur 4 eine Reformatierung eines Volumens im Rahmen eines Ausführungsbeispiels des erfindungsgemäßen Verfahrens,

Figur 5 die bei einer Bildung von Offsetflächen auftretenden Überschneidungen von Flächenelementen,

Figur 6 beispielhaft das Phänomen der Winkelverzerrung und Längenverzerrung bei der Reformatierung eines Volumens,

Figur 7 eine Benutzeroberfläche, welche ein reformatiertes zweidimensionales Bild eines zu untersuchenden dreidimensionalen Objekts als Navigationsfläche und zusätzlich verschiedene Ansichten des dreidimensionalen Objekts umfasst,

Figur 8 die Festlegung einer Position und des Sichtwinkels einer Kamera anhand von Normalen auf ein von der parametrisierten dreidimensionalen Fläche abgeleitetes Vektorfeld,

Figur 9 eine CPRVisualisierung auf Basis einer reformatierten zweidimensionalen Darstellung,

Figur 10 eine Vorrichtung zum zweidimensionalen Abbilden eines zu untersuchenden dreidimensionalen Objekts.

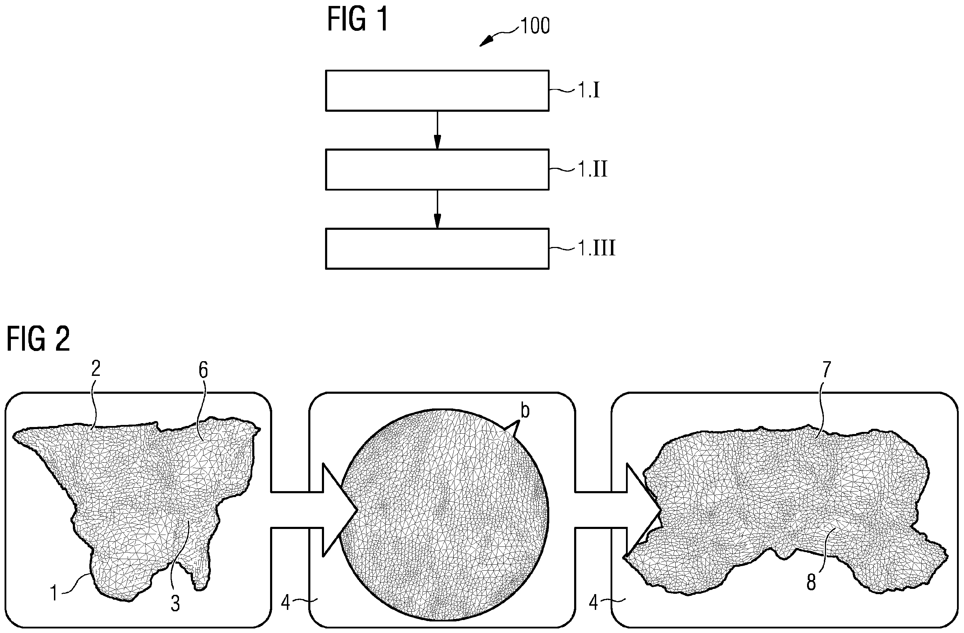

In Figur 1 sind die einzelnen Schritte des Verfahrens 100 zum Abbilden eines zu untersuchenden dreidimensionalen Objekts 1 in einem Flussdiagramm veranschaulicht. Bei dem Schritt 1.I wird eine dreidimensionale parametrisierte Fläche 2 festgelegt, welche konform zu der anatomischen Struktur des zu untersuchenden dreidimensionalen Objekts 1 ist. Bei dem Schritt 1.II wird die dreidimensionale parametrisierte Fläche 2 auf eine zweidimensionale parametrisierte Fläche 4 abgebildet. Schließlich wird bei dem Schritt 1.III das zu untersuchende dreidimensionale Objekt 1 durch Abbilden von der dreidimensionalen parametrisierten Fläche 2 zugeordneten Bildpunkten 5 auf die zweidimensionale parametrisierte Fläche 4 zweidimensional dargestellt.

In Figur 2 sind die Verfahrensschritte, welche die anatomiegesteuerte Reformatierung eines zu untersuchenden Objekts, in diesem Fall ein Beckenknochen, gemäß einem ersten Ausführungsbeispiel der Erfindung umfassen, veranschaulicht. In der linken Teilabbildung ist gezeigt, wie ein zwischen den Oberflächen des Beckens 1 zentriertes offenes Flächennetz 3 aus Dreiecken 6 festgelegt wird, wobei einige Randknotenpunkte bzw. Randkanten des Flächennetzes markiert sind. Dabei wird das dreidimensionale Flächennetz 3 durch eine Liste von Koordinaten $v_i \in R^3$, wobei i = 0...n1 ist, eine Liste von Dreiecken T und eine Liste von Halbkanten HE definiert. Wie bereits erwähnt, ist das Flächennetz ein offenes Netz mit einer Berandung.

In der mittleren Teilabbildung der Figur 2 ist gezeigt, wie das dreidimensionale Flächennetz auf eine initiale zweidimensionale Fläche 4 abgebildet wird. Dabei wird als initiale zweidimensionale Fläche eine Kreisfläche gewählt, auf deren Kreislinie b die Randknotenpunkte bzw. Randkanten des Flächennetzes abgebildet werden. Die Randknotenpunkte dienen als Randbedingungen für die Berechnung der übrigen Knotenpunkte $v_i'$ des zweidimensionalen Netzes. Die Verwendung einer Kreisfläche als Initialfläche ist nur der Einfachheit halber gewählt und es können auch alternativ andere Initialflächen gewählt werden. Zur Bestimmung der übrigen Knotenpunkte $v_i'$ auf der initialen zweidimensionalen Fläche wird folgendes diskretes lineares PoissonGleichungssystem berechnet:

$$\# V(i)v_i' - \sum_{j \in V(i) \cap \overline{\Omega}} v_j' = \sum_{j \in V(i) \cap \Omega} v_j' \qquad (1)$$

Dabei repräsentieren die $v_i'$ alle freien Knotenpunkte, welche nicht auf der Kreislinie $\Omega$ (in der Figur 2 auch mit b bezeichnet) liegen. V(i) bezeichnet den Satz von Nachbarknotenpunkten eines Knotenpunktes $v_i'$ und #V(i) die Größe des Satzes von Nachbarknotenpunkten, d.h. die Anzahl der Valenzen. Die mit j indizierten Knotenpunkte $v_j'$ sind die zu dem Knotenpunkt $v_i'$ benachbarten Knotenpunkte. Dies können bereits festgelegte Knotenpunkte auf dem Rand, d.h. der Kreislinie, sein, welche auf der rechten Seite der Gleichung 1 stehen, oder freie benachbarte Knotenpunkte sein, welche nicht auf der Kreislinie liegen und in der Gleichung 1 der Summe links von dem Gleichheitszeichen zugeordnet sind.

Von dieser Initialfläche bzw. diesem Initialnetz ausgehend wird nun ein Optimierungsprozess durchgeführt, wobei zunächst in einem lokalen Optimierungsschritt für jedes Dreieck eine 2DRotation berechnet wird, welche die Rotation $R_t$ des Dreiecks zwischen der 3DDarstellung und der einer idealen, unverzerrten, anschließend einem globalen iterativen Optimierungsprozess unterliegenden, Anordnung des Dreiecks definiert.

Für die Abbildung der Dreiecke von der 3DDarstellung auf die Initialfläche wird zunächst eine die Form des Dreiecks erhaltende Abbildung $N_t : R^3 > R^2$ definiert.

$$N_t(x) = \left( \frac{v_b - v_a}{\|v_b - v_a\|} \quad \frac{n \times (v_b - v_a)}{n \times \|v_b - v_a\|} \right)^T \left( x - \frac{1}{3}(v_a + v_b + v_c) \right) \qquad (2)$$

wobei

$$n = \frac{(v_c - v_a) \times (v_b - v_a)}{\|(v_c - v_a) \times (v_b - v_a)\|}$$

Dabei sind a, b und c die Indizes der Knotenpunkte eines Dreiecks $t_t$.

Zur Berechnung der Drehung zwischen dem auf die 2DDimension projizierten Dreieck $t^0_t$, wobei

$$t^0_t = \left( v_a^0, v_b^0, v_c^0 \right)_i = \left( N_t(v_a), N_t(v_b), N_t(v_c) \right) \qquad (3)$$

und dem Dreieck der aktuellen Lösung des Optimierungsverfahrens ($v'_a$, $v'_b$, $v'_c$) kann zum Beispiel der KabschAlgorithmus verwendet werden, welcher in W. Kabsch "A - solution for the best rotation to relate two sets of vectors"; Acta Crystallographica Section A, 32(5):922-923, 1976, erläutert wird. Dafür wird zunächst eine Einzelwertzerlegung A = VSW$^T$ der kovarianten Matrix A der Knotenpunkte relativ zu dem Schwerpunkt der aktuellen Dreiecke berechnet.

$$A = v_a^0\left(v'_a - c\right)^T + v_b^0\left(v'_b - c\right)^T + v_c^0\left(v'_c - c\right)^T \qquad (4)$$

Dabei gibt c = 1/3($v'_a$+$v'_b$+$v'_c$) den Schwerpunkt der aktuellen dem Optimierungsverfahren zugrundeliegenden Dreiecke an.

Die Rotation $R_t$ zwischen den ursprünglichen unverzerrten aber bereits auf die 2DFläche projizierten Dreiecken und den aktuellen dem Optimierungsverfahren zugrundeliegenden Dreiecken ergibt sich dann zu

$$R_t = W\begin{pmatrix} 1 & 0 \\ 0 & sign\left(\det\left(WV^T\right)\right) \end{pmatrix} V^T \qquad (5)$$

In einem globalen Optimierungsschritt, nachfolgend auch als globale Phase bezeichnet, müssen die einzelnen gedrehten Dreiecke in der zweidimensionalen Darstellung für die gefundenen optimalen Rotationen $R_t$ wieder verbunden werden, so dass eine zusammenhängende Fläche entsteht. Die gesuchten Positionen der Knotenpunkte $v'_i$ ergeben sich aus dem folgenden optimierten Energiefunktional:

$$E_{ARAP}\left(v'_i, R\right) = \frac{1}{2}\sum_{(i,j)\in HE}\cot\left(\Theta_{i,j}\right)\left\|\left(v'_i - v'_j\right) - R_{t(i,j)}\left(v_i^0 - v_j^0\right)\right\|^2 \qquad (6)$$

Dabei sind cot($\theta_{i,j}$) kotangentiale Gewichte, wie sie in U. Pinkall and K. Polthier "Computing discrete minimal surfaces and their conjugates" Experimental mathematics, 2(1):15-36, 1993, beschrieben werden. HE sind Sätze von Halbkanten in dem Netz, $R_{t(i,j)}$ ist die Rotation eines Dreiecks mit der Halbkante (i, j), $v_i^0 \in \Re^3$ sind die Knotenpunktpositionen der ursprünglichen Dreiecke und $v'_i \in \Re^2$ sind die zu optimierenden Knotenpunkte. Zur Erleichterung der Berechnung der Zielknotenpunkte können die in dem lokalen Optimierungsprozess ermittelten Rotationen $R_{t(i,j)}$ festgehalten werden, damit bei der Optimierung ein lineares Gleichungssystem entsteht. Anders ausgedrückt, werden die Drehungen $R_t$ und die Knotenpunkte $v'_i$ in separaten Schritten optimiert. Nachdem das Gleichungssystem (6) gelöst wurde, also die Knotenpunkte $v'_i$ ermittelt wurden bzw. optimiert wurden, werden nun die entsprechenden Drehungen $R_{t(i,j)}$ neu berechnet und anschließend der Optimierungsschritt der Knotenpunkte wiederholt, so dass sich ein iteratives Optimierungsverfahren ergibt.

Bei der Optimierung wird der Gradient der Energie $E_{ARAP}$ gleich 0 gesetzt. Es ergibt sich somit folgendes lineares Gleichungssystem:

$$\sum_{j\in V(i)}\left(\cot\left(\Theta_{i,j}\right) + \cot\left(\Theta_{j,i}\right)\right)\left(v'_i - v'_j\right) = \sum_{j\in V(i)}\left(\cot\left(\Theta_{i,j}\right)R_{t(i,j)} + \cot\left(\Theta_{j,i}\right)R_{t(j,i)}\right)\left(v_i^0 - v_j^0\right) \qquad (7)$$

wobei i = 1.... N1.

Zusammengefasst ist das Optimierungsverfahren durch eine lokale Phase mit einem Berechnen von Rotationen zwischen den ursprünglichen bzw. den optimierten Dreiecken und den Dreiecken der jeweiligen aktuellen iterativen Lösung gekennzeichnet und durch eine globale Phase, bei der ein Energieterm, der dem Netz mit optimal rotierten Dreiecken zugeordnet ist, minimiert wird. Die beiden die lokale Phase und die globale Phase umfassenden Schritte werden in einem iterativen Verfahren alternierend immer wieder ausgeführt. Der Ansatz mit einer lokalen und einer globalen Phase hat den Vorteil, dass in der globalen Phase die Rotationen fix sind, also keine Unbekannten. Dass bedeutet, dass das Optimierungsproblem linear wird und mit Hilfe von Matritzen, also in einem linearen Gleichungssystem, beschrieben werden kann. Die Optimierung muss allerdings nicht zwangsläufig so von statten gehen. Man könnte auch direkt das Energiefunktional der Gleichung 6 minimieren.

Das Ergebnis dieses iterativen Prozesses ist in der rechten Teilabbildung der Figur 2 gezeigt. Es ist ein zweidimensionales Netz 7, dessen Netzelemente 8 eine optimale Ähnlichkeit zu den Netzelementen 6 des dreidimensionalen Flächennetzes 3 aufweisen.

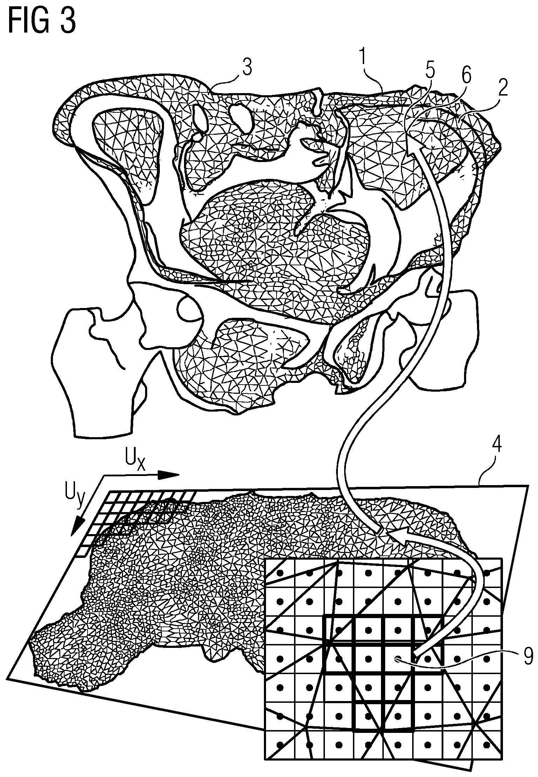

Bei dem ersten Ausführungsbeispiel des erfindungsgemäßen Verfahrens folgt als nächstes ein Abtastschritt, wobei jedem der Pixel in dem zweidimensionalen Netz ein Bildwert bzw. ein Intensitätswert der entsprechenden Position in dem dreidimensionalen Flächennetz zugeordnet wird. Diese Vorgehensweise ist in der Figur 3 verdeutlicht. Dieser Verfahrensschritt wird auch als Resampling bezeichnet. Bei dem Resampling wird, nachdem jede der optimalen Knotenpunktpositionen $v'_i$ berechnet wurde, in Verbindung mit dem ursprünglichen dreidimensionalen Flächennetz ein punktweises Abtasten des dreidimensionalen Flächennetzes und Abbilden auf die Pixel der zweidimensionalen Abbildung realisiert. Um aus dem zweidimensionalen Netz ein Bild, im Folgenden auch ADRBild genannt, mit den Eigenschaften des dreidimensionalen Bilds mit einer bestimmten Auflösung $ADR_{resx}*ADR_{resy}$ zu erzielen, wird zunächst die Ausdehnung des ADRBildes mit Grenzwerten $v'_{min}$ und $v'_{max}$ in x und yRichtung festgelegt. Auf dieser Basis können Koordinaten $u_i$ des ADRBildes bestimmt werden:

$$ u_i = \begin{pmatrix} u_{ix} \\ u_{iy} \end{pmatrix} = \begin{pmatrix} \left(v'_{ix} - v'_{minx}\right)\dfrac{ADR_{resx}}{v'_{maxx} - v'_{minx}} \\ \left(v'_{iy} - v'_{miny}\right)\dfrac{ADR_{resy}}{v'_{maxy} - v'_{miny}} \end{pmatrix} \qquad (8) $$

Zur Bestimmung der Intensität der Pixel des ADRBilds wird zunächst das entsprechende Dreieck in dem ADRBild ermittelt, in dem der entsprechende Pixel positioniert ist. Die baryzentrischen Koordinaten des Pixels werden berechnet und die entsprechende Position in dem 3DFlächennetz berechnet. Das Abtasten des Volumens an der entsprechenden Position in der 3DDarstellung ergibt dann den Intensitätswert für den zugeordneten Pixel. Zur Beschleunigung der Suche nach dem Dreieck, dem der Pixel zugeordnet ist, kann auch eine LookupTabelle berechnet werden. In der LookupTabelle werden jedem Pixel alle Dreiecke zugeordnet, die das Pixel überlappen (bei feinen Dreiecksgittern können lokal die Dreiecke kleiner sein als die Pixel im Zielbild und andersherum. Baryzentrische Koordinaten müssen dann nur für diese Dreiecke (und nicht für alle Dreiecke) berechnet werden.

Bei dem in den Figuren 2 und 3 veranschaulichten ersten Ausführungsbeispiel wird nur ein reformatiertes 2DBild einer einzigen Schnittfläche erzeugt. Allerdings ist es bei der Untersuchung von volumetrischen medizinischen Datensätzen oft notwendig, die Umgebung eines bestimmten Bereichs genauer zu inspizieren, indem benachbarten Schichten des rekonstruierten Bildes betrachtet werden. Um auch diese Art der medizinischen Untersuchung zu beschleunigen und zu flexibilisieren, kann auch das in den Figuren 2 und 3 skizzierte Verfahren angewandt werden. In diesem Fall ist das Verfahren nicht auf die Visualisierung einer einzelnen Fläche der 3DDarstellung beschränkt, sondern es wird auch die Umgebung bzw. das umgebende Volumen einer parametrisierten Fläche mit einbezogen.

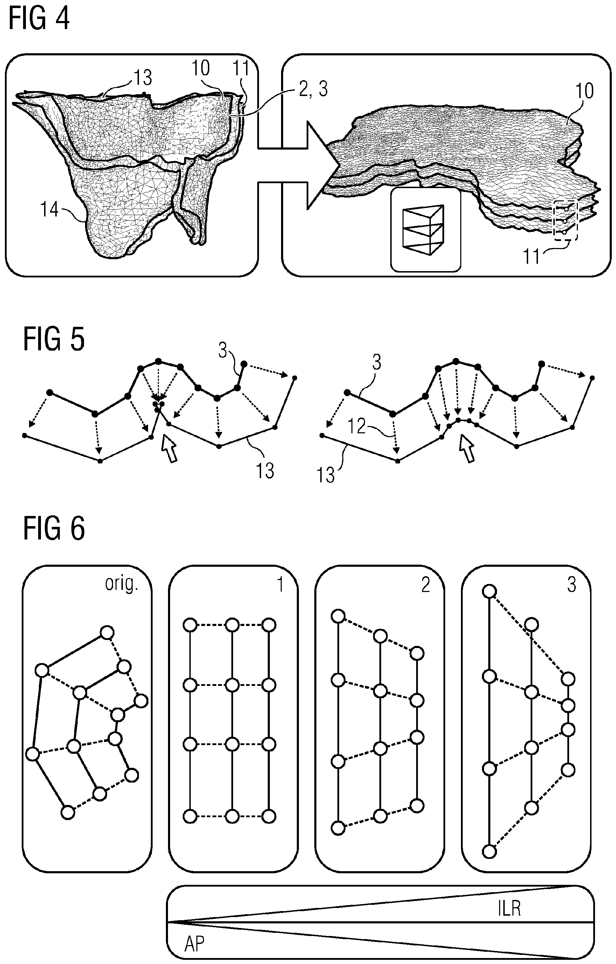

Bei einem zweiten Ausführungsbeispiel wird die Umgebung einer wie in der Figur 2 parametrisierten Fläche parametrisiert, indem um die bereits parametrisierte Fläche sogenannte Offsetflächen 10, 11 bzw. OffsetFlächennetze 13, 14 berechnet werden. Im einfachsten Fall wird dafür auf beiden Seiten der bereits parametrisierten Fläche eine Offsetfläche 10, 11 bzw. ein OffsetFlächennetz 13, 14 berechnet. Diese Vorgehensweise ist in Figur 4 veranschaulicht. Die dort gezeigten OffsetFlächennetze 13, 14 sind beispielsweise einfache Kopien des bereits parametrisierten dreidimensionalen Netzes 3 der bereits parametrisierten Zentralfläche 2 in positiver und negativer Richtung einer Normalen relativ zu der parametrisierten Fläche. Die Knotenpunkte haben folglich die Position $v^-=v$ dn und $v^+= v + dn$, wobei d ein festgelegter Abstand der Offsetflächen zu der Zentralfläche ist und n die Normale auf die Zentralfläche an der Position der Knotenpunkte v darstellt. Wenn Reformatierungen mit konstanter Dicke erzeugt werden sollen, so wird d über die gesamte parametrisierte Fläche bzw. über die gesamte ADRFläche als konstant festgelegt, so dass bei der Reformatierung statt einer einfachen zweidimensionalen ADRFläche ein Stapel aus drei ADRFlächen entsteht, wie es in der rechten Teilzeichnung in Figur 4 gezeigt ist.

Bei der einfachsten Variante der zweiten Ausführungsform wird also lediglich die mittlere dreidimensionale parametrisierte Fläche auf eine 2DFläche projiziert und die beiden OffsetFlächennetze 13, 14 weisen dieselbe Geometrie auf wie die zentrale Fläche. Diese Vorgehensweise ist in der linken Teilzeichnung der Figur 5 verdeutlicht. Der Abtastschritt ist in diesem Fall besonders einfach, weil die Netzstruktur in den verschiedenen Offsetnetzen 13, 14 jeweils identisch ist. Allerdings treten bei diesem Ansatz häufig sogenannte Selbstüberschneidungen auf, wenn die versetzen OffsetNetze 13, 14 für die Parametrisierung der versetzten Offsetflächen berechnet werden. Genauer gesagt, schneiden sich die Normalen 12, mit denen die Offsetnetze definiert werden sollen. Dieses Phänomen ist ebenfalls in der linken Teilzeichnung in Figur 5 gezeigt. Um die Selbstüberschneidungen zu reduzieren, können die Positionen der Knotenpunkte der Offsetnetze derart korrigiert werden, dass sie nicht mehr auf den Normalen 12 der Knotenpunkte der dreidimensionalen ADRFläche liegen, was in der rechten Teilzeichnung in Figur 5 gezeigt ist. Allerdings sind damit bei der zweidimensionalen Darstellung Verzerrungen verbunden.

Da die ADRFläche (die dreidimensionale parametrisierte Fläche) in der Regel nicht planar ist, unterscheidet sich die Flächengröße der Dreiecke der Offsetflächen von den entsprechenden Dreiecken der ADRFläche, wie es in Figur 6 in der linken, mit orig. gekennzeichneten Teilzeichnung erkennbar ist. Der Umfang der Verkleinerung oder Vergrößerung hängt ab von der Krümmung der ADRFläche 2 an der entsprechenden Stelle und ist unterschiedlich über das gesamte Netz. Somit bringt das Verwenden derselben Geometrie für alle ADRFlächen, wie es bei dem Ausführungsbeispiel in der Figur 5 und bei der in Figur 6 mit der Nummer 1 bezeichneten Teilzeichnung der Fall ist, eine Volumenverzerrung mit sich, so dass lokal keine Längentreue ILR vorherrscht. Um der unterschiedlichen Größe der Dreiecke in den unterschiedlichen Offsetflächen Rechnung zu tragen, werden die zweidimensionalen Netze der unterschiedlichen Schichten in der reformatierten Darstellung derart modifiziert, dass sie nun unterschiedlich dimensionierte Dreiecke mit unterschiedlichen Knotenpunkten haben. Anders ausgedrückt, wird nun ein gemeinsames dreidimensionales Netz auf alle Schichten ausgedehnt und die lokale und globale Reformatierung nun für alle Schichten ausgeführt. Das zugehörige zweidimensionale Netz ergibt sich wie folgt:

$$v' \leftarrow \left[ v'_0 ... v'_{n-1} , v'_n ... v'_{2n-1} , v'_{2n} ... v'_{3n-1} \right] \qquad (9)$$

Dabei sind die Indizes 0 bis n1 beispielsweise der unteren Schicht eines Stapels von Schichten zugordnet, die Indizes n bis 2n1 der mittleren Schicht und 2n bis 3n1 der oberen Schicht eines Schichtstapels zugeordnet. Die v' sind Knotenpunkte analog zu denen des Netzes der Gleichung 1.

Entsprechend wird die topologische Information betreffend die reformatierten Dreiecke T' und die reformatierten Halbkanten HE' erweitert. Damit geht eine Erweiterung der Anzahl der Unbekannten des Gleichungssystems 7 auf das Dreifache einher. Die einzelnen Dreiecke der Offsetflächen werden somit mit größtmöglicher Längentreue ILR in der zweidimensionalen Darstellung abgebildet. Dies ist in der mit der Nummer 3 bezeichneten Teilzeichnung der Figur 6 veranschaulicht. Allerdings wird die Längentreue mit einer geringeren Winkeltreue AP erkauft, so dass sogenannte Schereffekte auftreten.

Um einen Kompromiss zwischen diesen beiden Extremen zu erzielen, kann dem Energiefunktional der Gleichung 6 ein zusätzlicher Scherterm hinzugefügt werden. Ein Ziel dabei besteht darin, die Knotenpunkte $V_i$ der versetzten Schichten und der Mittenschicht an bezüglich des lokalen Tangentenraums ähnlichen Relativpositionen zu halten. Dafür wird zunächst für jeden Knotenpunkt einzeln für jede Offsetfläche ein Offset $o_i^-$ bzw. $o_i^+$ ermittelt. Zur Berechnung dieses Offsets werden zunächst die Knotenpunkte $v_i^-$ bzw. $v_i^+$ in den Tangentenraum der ADRFläche bei dem zugeordneten Knotenpunkt $V_i$ transformiert. Da die Transformationen $N_t$ nur für Dreiecke definiert sind und im Allgemeinen mehrere Dreiecke an einem Knotenpunkt anliegen, werden die von den verschiedenen Dreiecken $t \in T(i)$ beigetragenen Offsets gemittelt, um einen gemittelten Offset $o_i^-$ bzw. $o_i^+$ jedes Knotenpunkts zu erhalten. Die gemittelten Offsets errechnen sich also wie folgt:

$$o_i^- = \frac{1}{\#T(i)} \sum_{t \in T(i)} R_t \left( N_t \left( v_i^- \right) \right)$$

$$\forall i = 0 ... n - 1 \qquad (10)$$

$$o_i^+ = \frac{1}{\#T(i)} \sum_{t \in T(i)} R_t \left( N_t \left( v_i^+ \right) \right)$$

wobei $T(i)$ ein Satz von Dreiecken ist, welche an dem Knotenpunkt $V_i$ der Mittenschicht anliegen. $\#T(i)$ umfasst die Anzahl der Dreiecke des Satzes von Dreiecken. Die beiden Offsets $o_i^-$ bzw. $o_i^+$ hängen nur von der Geometrie der initialen ADRFläche ab und sind während der Optimierung der ADRFläche bzw. der entsprechenden Flächenstapel konstant.

Die unterschiedlichen Offsets werden nun auch bei dem Energieterm der Gleichung 6 berücksichtigt, wobei der bereits erwähnte Scherterm hinzugefügt wird. Die hieraus sich ergebende Volumenenergie errechnet sich wie folgt:

$$E_{VOL}^\alpha \left( v_i', R \right) = E \left( v_i', R \right) + \alpha E_{shear} \left( v_i', R \right) \qquad (11)$$

dabei setzt sich der Scherterm $E_{shear}$ wie folgt zusammen:

$$E_{shear}(v'_i, R) = \sum_{i=0...n-1} \left\| (v'_{i+n} - v'_i) - o_i^- \right\|^2 + \left\| (v'_{i+n} - v'_{i+2n}) - o_i^+ \right\|^2 \qquad (12)$$

Die Gewichtung $\alpha$ erlaubt es, den Einfluss des Scherterms zu verändern. Gleichung 11 beschränkt in Verbindung mit Gleichung 12 die Knotenpunkte der Offsetschichten auf Offsets, die ähnlich denen des undeformierten Zustands sind. Somit wird eine übermäßige Winkelverzerrung, d.h. Scherung, der Offsetschichten relativ zueinander vermieden. Ein größeres $\alpha$ führt zu einem formatierten Volumen mit einer geringeren Winkelverzerrung aber einer umso größeren Längenverzerrung und umgekehrt. Ein Kompromiss zwischen Winkelverzerrung und Längentreue wird also bei einem mittleren Wert für $\alpha$ erreicht, was durch die mit 2 gekennzeichnete Teilzeichnung der Figur 6 veranschaulicht ist. Die positive und die negative Schicht sind beide um eine bestimmte vorher festgelegte Distanz d zu der mittleren Schicht verschoben.

Bei dem Schritt des Abtastens muss der Umstand berücksichtigt werden, dass die Berechnung der baryzentrischen Koordinaten nun von der zPosition, d.h. der Position in OffsetRichtung des jeweiligen Dreiecks abhängig ist. Daher muss in diesem Fall eine Interpolation durchgeführt werden. Genauer gesagt, wird eine Art lineares Mischen angewandt. Ein Dreieck bei einer bestimmten Höhe z ist daher wie folgt definiert:

$$\begin{aligned} T(z) = {} & u * (v'_{a-n}, v'_{b-n}, v'_{c-n}) \\ & + v * (v'_a, v'_b, v'_c) \\ & + w * (v'_{a+n}, v'_{b+n}, v'_{c+n}), \quad z \in [0, 2d] \end{aligned} \qquad (13)$$

wobei

$$u = \begin{cases} \left(1 - \dfrac{z}{d}\right) & 0 < z < d \\ 0 & d < z < 2d \end{cases}$$

$$v = \begin{cases} \dfrac{z}{d} & 0 < z < d \\ 1 - \dfrac{z-d}{d} & d < z < 2d \end{cases}$$

$$w = \begin{cases} 0 & 0 < z < d \\ \dfrac{z-d}{d} & d < z < 2d \end{cases}$$

Dabei sind $(v'_{an}, v'_{bn}, v'_{cn})$, $(v'_a, v'_b, v'_c)$, $(v'_{a+n}, v'_{b+n}, v'_{c+n})$ die Instanzen eines Dreiecks in der negativen bzw. unteren, mittleren und der positiven bzw. oberen ADRSchicht. Da Dreiecke nun in Abhängigkeit von z unterschiedliche Pixel umfassen, wird eine Art Mischung der von den Halbkanten eines Dreiecks umschlossenen Bereiche in allen Schichten vorgenommen, wenn sie in einer LookupTabelle zum Abtasten aufgelistet werden.

Wie in den vorhergehenden Abschnitten erläutert, lassen sich Verzerrungen in der zweidimensionalen Abbildung zwar reduzieren aber grundsätzlich nicht verhindern. Da jedoch einige Bereiche der ADRBildfläche wichtiger sind als andere, kann es von Vorteil sein, dieses bereits bei der Reformatierung zu berücksichtigen. Für diesen Zweck ist es sinnvoll, Wichtigkeitskarten zu verwenden, mit denen die Verteilung der Verzerrungen während der Reformatierung gesteuert werden kann. Damit können die Fehler bzw. Verzerrungen in bestimmten Bereichen mit größerer Wichtigkeit verringert werden, wofür im Gegenzug in den Bereichen mit geringerer Priorität ein höheres Ausmaß an Verzerrungen akzeptiert wird.

Um die Verzerrungen bei der Reformatierung steuern zu können, wird entsprechend der Wichtigkeit eines Bereiches jedem der Knotenpunkte $v_i$ ein Gewicht $w_i$ zugewiesen. Diese Zuweisung kann beispielsweise vorab geschehen, indem in einem ADRReferenznetz bestimmten Bereichen bestimmte Gewichte zugewiesen werden. Alternativ kann die Zuweisung der Gewichte auch interaktiv in einer reformatierten Darstellung geschehen, wobei anschließend der Reformatierungsprozess erneut gestartet wird. Beispielsweise können bei der Darstellung von Skelettteilen Masken für die Skelettbereiche durch Festlegen von Schwellwerten und Nachbearbeiten des Ausgangsbildes erzeugt werden. Diese Masken können direkt verwendet werden, um die Gewichte den Knotenpunkten einer ADRFläche zuzuordnen, wodurch in den Bereichen mit den Skelettteilen die Verzerrungen reduziert werden.

Um die Gewichte bei der Reformatierung mit zu berücksichtigen, müssen sie in die Berechnung der Energie nach Gleichung 6 bzw. in das Gleichungssystem 7 mit einfließen. Dabei wird jeder Term in der Gleichung 6 mit dem Gewicht der jeweiligen Halbkante $w_{(i,j)}$ multipliziert, wobei diese Gewichte den Durschnitt $w_{(i,j)} = 0{,}5*(w_i+w_j)$ der Gewichte $w_i$ und $w_j$ der zu der jeweiligen Halbkante gehörenden Knotenpunkte repräsentieren. Das Gleichungssystem unter Berücksichtigung der Scherenergie $E_{shear}$ und der Gewichte $w_{(i,j)}$ ergibt sich dann wie folgt:

$$\sum_{j\in V(i)} w_{(i,j)}\big(\cot(\Theta_{i,j})+\cot(\Theta_{j,i})\big)\big(v'_i - v'_j\big) + A_i$$
$$= \sum_{j\in V(i)} w_{(i,j)}\big(\cot(\Theta_{i,j})R_{t(i,j)}+\cot(\Theta_{j,i})R_{t(j,i)}\big)\big(v^0_i - v^0_j\big) + B_i \qquad (14)$$
$$\forall i = 0...3n-1$$

$A_i$ und $B_i$ ergeben sich aus dem Scherterm und verbinden die einzelnen ADRSchichten:

$$A_i = \begin{cases} v'_i - v'_{n+i} & i < n \\ 2v'_i - v'_{i-n} - v'_{i+n} & n < i < 2n \\ v'_i - v'_{i-n} & 2n < i < 3n \end{cases} \qquad (15)$$

$$B_i = \begin{cases} -o^-_i & i < n \\ o^-_{i-n} + o^+_{i-n} & n < i < 2n \\ -o^+_{i-2n} & 2n < i < 3n \end{cases} \qquad (16)$$

Die linke Seite der Gleichung 14 weist konstante Koeffizienten auf und das entsprechende lineare System ist dünn besetzt und symmetrisch. Die Koeffizienten können also einmal festgelegt und dann während der Iterationen beibehalten werden. Dagegen müssen die Rotationen $R_t$ auf der rechten Seite bei jeder Iteration erneut berechnet werden, da sie von der Geometrie der aktuellen Lösung abhängen.

Das beschriebene Ausführungsbeispiel, welches ein Abbildungsverfahren mit drei Schichten im Detail veranschaulicht, kann auf beliebig viele Schichten erweitert werden. Bei einer größeren Anzahl von abgebildeten Schichten werden die Verzerrungen durch das optimierte Gitter noch feiner minimiert. Die Anzahl der OffsetFlächen kann also beliebig gewählt werden. Die Verbindungen bzw. Kanten und der Energieterm gemäß Gleichung 12 lassen sich ohne zusätzliche Schwierigkeiten auf beliebig viele Schichten verallgemeinern.

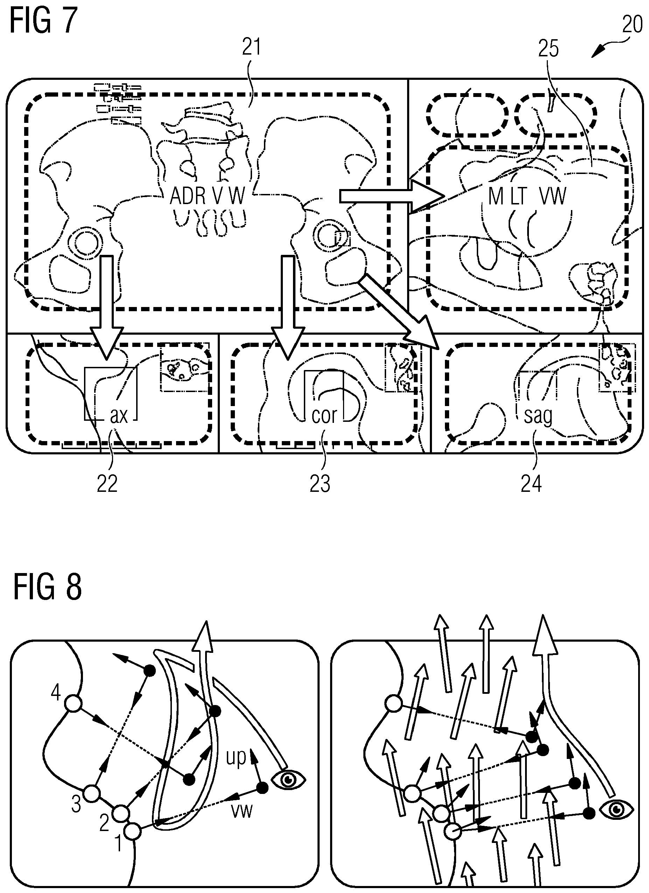

Im Gegensatz zu den Ansätzen gemäß dem Stand der Technik kann das erfindungsgemäße Verfahren für eine große Bandbreite unterschiedlicher anatomischer Strukturen eingesetzt werden. Zwar sind die ausführlich beschriebenen Ausführungsbeispiele auf Skelettteile beschränkt, jedoch ist das Verfahren auch auf gänzlich andersartige anatomische Strukturen anwendbar. Um die erzeugten ADRBilder effektiv nutzen zu können, ist es sinnvoll, diese Bilder in eine geeignete medizinische Visualisierungsumgebung einzuordnen. Eine geeignete Benutzeroberfläche 20 für diese Visualisierungsumgebung ist in Figur 7 gezeigt. Die Visualisierungsumgebung ist derart aufgeteilt, dass links oben das ADRBild 21, auch mit ADRVW (ADR View) bezeichnet, positioniert ist, während im unteren Bereich Schnittbilder der Bildaufnahme zu sehen sind. Das Schnittbild 22 ist eine Darstellung in axialer Richtung, welche in der Figur 7 auch mit ax gekennzeichnet ist. Das Schnittbild 23 ist eine Darstellung in koronaler Richtung, die in Figur 7 mit cor bezeichnet ist, und das Schnittbild 24 ist eine Darstellung in sagittaler Richtung, die in Figur 7 mit sag bezeichnet ist. Rechts oben ist eine perspektivische Ansicht 25, auch mit MLTVW (Multi View) bezeichnet, zu sehen. In dieser Visualisierungsumgebung wird das ADRBild 21 zur Navigation in den anderen Bildern verwendet, weil in dem ADRBild die Übersicht für den Benutzer am ehesten gegeben ist. Die unterschiedlichen Ansichten sind miteinander derart verbunden, dass in Abhängigkeit von der Position in der Ansicht in dem ADRBild 21 auch die anderen Ansichten wechseln. Verschiebt man zum Beispiel die Position des in dem ADRBild rechts unterhalb der Bildmitte angeordneten Quadrats, welches eine Position der Detailansichten 22 bis 24 festlegt, so ändern sich entsprechend auch die Ansichten 22 bis 24. Die in den Ansichten 22 bis 24 zentral angeordneten Quadrate entsprechen dem von dem Quadrat in dem ADRBild festgelegten Bildausschnitt.

Ändert der Betrachter die Position in dem ADRBild, so ändert sich der Betrachtungswinkel für die perspektivische Ansicht 25, es wird also quasi die Kameraperspektive geändert, wobei die Position der virtuellen Kamera, die Sichtrichtung und die Kamerarotation bei einem dynamischen Vorgang eine Rolle spielen. Ein solches Szenario ist in der Figur 8 veranschaulicht. Um die Sichtrichtung vw in der perspektivischen Ansicht zu berechnen, kann die entsprechende Normale auf das dreidimensionale Flächennetz verwendet werden. Dabei kann der positive Normalenvektor als Richtung der Kameraposition definiert werden und der negative Normalenvektor als Sichtrichtung vw festgelegt werden. Aufgrund der Krümmung der ADRFlächen führt ein Ausrichten der Kameraposition anhand der Normalen der ADRFlächen zu einer unstetigen Kamerabewegung, wie sie in Figur 8 in der linken Teilzeichnung veranschaulicht ist. In Figur 8 ist zusätzlich zur Sichtrichtung vw noch die senkrecht dazu orientierte Aufwärtsrichtung up des Betrachters bzw. der Kamera eingezeichnet. Um eine kontinuierlichere Kamerabewegung zu erreichen, wird mit vertexskinningTechniken ein Vektorfeld längs des dreidimensionalen Flächennetzes definiert und während des Optimierungsprozesses des zweidimensionalen Netzes entsprechend mit angepasst. Die Normalen auf die mit den vertextskinning Techniken erzeugten Vektoren ergeben die beruhigte Kameraposition.

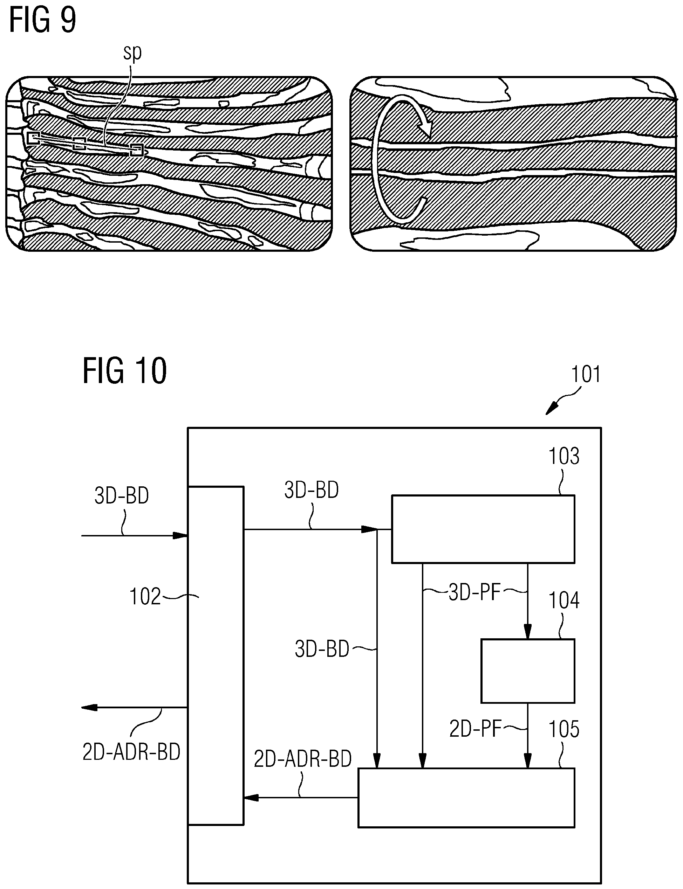

Eine zusätzliche Möglichkeit, eine verbesserte Ansicht zu erhalten, kann die Kombination der ADRBilder mit einer CPRVisualisierung darstellen, wie sie in Figur 9 gezeigt ist. Dabei wird zunächst in dem ADRBild ein Spline sp festgelegt, der den Verlauf eines Rotationszentrums definiert, um das herum eine CPRVisualisierung eines Teilbereichs des aufgenommenen Objekts durchgeführt werden soll. Der in dem ADRBild festgelegte Spline wird anschließend in den dreidimensionalen Bildraum der Aufnahme umgerechnet und kann dazu verwendet werden, um eine CPRVisualisierung um das festgelegte Zentrum zu realisieren.

In Figur 10 ist eine Vorrichtung 101 zum zweidimensionalen Abbilden eines zu untersuchenden dreidimensionalen Objekts gezeigt. Eine Schnittstelleneinheit 102 umfasst eine Eingangs und Ausgangsschnittstelle, welche die Bilddaten eines aufgenommenen 3DBildes 3DBD eines zu untersuchenden dreidimensionalen Objekts erfasst und die erstellten zweidimensionalen ADRBilddaten 2DADRBD ausgibt. Die erfassten 3DBilddaten 3DBD werden an eine FlächennetzErmittlungseinheit 103 übermittelt. Diese hat die Funktion, eine dreidimensionale parametrisierte Fläche festzulegen, welche konform zu der anatomischen Struktur des zu untersuchenden dreidimensionalen Objekts ist. Die Daten der dreidimensionalen parametrisierten Fläche 3DPF werden an eine Reformatierungseinheit 104 übermittelt, welche die dreidimensionale parametrisierte Fläche auf eine zweidimensionale parametrisierte Fläche abbildet. Die Daten der parametrisierten zweidimensionalen Fläche 2DPF sowie die übrigen Bilddaten 3DBD und 3DPF werden an eine Abtasteinheit 105 übermittelt. Diese hat die Funktion, das zu untersuchende dreidimensionale Objekt durch Abbilden von der dreidimensionalen parametrisierten Fläche zugeordneten Bildpunkten auf die zweidimensionale parametrisierte Fläche abzubilden. Die dabei erzeugten ADRBilddaten werden über die Schnittstelleneinheit 102 an beispielsweise eine Ausgabeeinheit oder eine Speichereinheit (nicht gezeigt) übermittelt.

Die beschriebenen Verfahren erlauben es dem Anwender, das bei medizinischen bildgebenden Verfahren erzeugte Bildmaterial schnell und effektiv auszuwerten. Dabei sind die ausführlich beschriebenen Verfahren bei sehr unterschiedlichen Untersuchungsobjekten flexibel einsetzbar, was den Aufwand für den Anwender reduziert und die Wirtschaftlichkeit des Einsatzes dieser Verfahren erhöht.

Es wird abschließend noch einmal darauf hingewiesen, dass es sich bei den zuvor beschriebenen detaillierten Verfahren und Aufbauten um Ausführungsbeispiele handelt und dass das Grundprinzip auch in weiten Bereichen vom Fachmann variiert werden kann, ohne den Bereich der Erfindung zu verlassen, soweit er durch die Ansprüche vorgegeben ist. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" oder "Modul" nicht aus, dass diese(s) aus mehreren Komponenten besteht, die gegebenenfalls auch räumlich verteilt sein können.

**Patentansprüche**

1. Von einem Computer ausgeführtes Verfahren (100) zum Abbilden eines zu untersuchenden dreidimensionalen Objekts (1), aufweisend die Schritte:

   - Festlegen einer dreidimensionalen parametrisierten Fläche (2), welche konform zu einer anatomischen Struktur des zu untersuchenden dreidimensionalen Objekts (1) ist,
   - Parametrisieren einer Umgebung der dreidimensionalen parametrisierten Fläche (2),
   - volumetrisches Deformieren der Umgebung der dreidimensionalen parametrisierten Fläche (2) in eine aufgefaltete Form, aufweisend einen Schichtstapel mit einer zweidimensionalen parametrisierten Fläche (4),
   - Darstellen des zu untersuchenden dreidimensionalen Objekts (1) in aufgefalteter Form durch Abbilden von der parametrisierten Umgebung der dreidimensionalen parametrisierten Fläche (2) zugeordneten Bildpunkten

**EP 3 155 597 B1**

(5) auf die zweidimensionale parametrisierte Fläche (4) des Schichtstapels.

2. Verfahren (100) nach Anspruch 1, wobei

    - die dreidimensionale parametrisierte Fläche (2) durch ein dreidimensionales Flächennetz (3) mit einer Vielzahl von Einzelelementen (6) parametrisiert wird,
    - die zweidimensionale parametrisierte Fläche (4) durch ein zweidimensionales Netz (7) mit einer Vielzahl von Einzelelementen (8) parametrisiert wird,
    - das zweidimensionale Darstellen des zu untersuchenden dreidimensionalen Objekts (1) durch Abbilden von den Einzelelementen (6) des dreidimensionalen Flächennetzes (3) zugeordneten Bildpunkten (5) auf die Einzelelemente (8) des zweidimensionalen Netzes (7) erfolgt.

3. Verfahren (100) nach Anspruch 1 oder 2, wobei die dreidimensionale parametrisierte Fläche (2) eine offene Fläche oder eine geschlossene Fläche ist, die vor der Abbildung der dreidimensionalen parametrisierten Fläche (2) auf eine zweidimensionale parametrisierte Fläche (4) in eine offene dreidimensionale parametrisierte Fläche (2) gewandelt wird.

4. Verfahren (100) nach einem der Ansprüche 2 oder 3, wobei die Einzelelemente (6, 8) Dreiecke und/oder Vierecke und/oder Sechsecke und/oder andere Vielecke umfassen und/oder das dreidimensionale Flächennetz (3) ein reguläres Netz oder ein irreguläres Netz ist.

5. Verfahren (100) nach einem der Ansprüche 1 bis 4, wobei der Schritt des Abbildens der dreidimensionalen parametrisierten Fläche (2) auf die zweidimensionale parametrisierte Fläche (4) durch Optimieren eines der dreidimensionalen parametrisierten Fläche (2) und einer zu optimierenden zweidimensionalen parametrisierten Fläche (4) zugeordneten Energieterms realisiert wird.

6. Verfahren (100) nach einem der Ansprüche 1 bis 5, wobei zusätzlich zu der dreidimensionalen parametrisierten Fläche (2) eine Mehrzahl von dreidimensionalen parametrisierten Offset-Flächen (10, 11), welche konform zu der anatomischen Struktur des zu untersuchenden dreidimensionalen Objekts (1) sind, erzeugt werden und jeweils auf eine zweidimensionale parametrisierte Fläche abgebildet werden, so dass das dreidimensionale Objekt auf einen dreidimensionalen Schichtstapel aus zweidimensionalen Flächen abgebildet wird.

7. Verfahren (100) nach einem der Ansprüche 1 bis 6, wobei die dreidimensionalen parametrisierten Offset-Flächen (10, 11) durch Ermitteln von zu der dreidimensionalen parametrisierten Fläche (2) orthogonalen Normalenvektoren (12) festgelegt werden.

8. Verfahren (100) nach einem der Ansprüche 1 bis 7, wobei die dreidimensionalen parametrisierten Offset-Flächen (10, 11) Offset-Flächennetze (13, 14) umfassen, die durch Anwendung von Gitternetz-Glättungsverfahren geglättet werden, wobei Überschneidungen von benachbarten Normalenvektoren (12) vermieden werden.

9. Verfahren (100) nach Anspruch 7, wobei die dreidimensionalen Offset-Flächennetze (13, 14) explizit berechnet werden.

10. Verfahren (100) nach einem der Ansprüche 6 bis 9, wobei mit dem Energieterm die Längentreue innerhalb der Schichten und die Winkelverzerrung zwischen den Schichten separat modelliert wird.

11. Verfahren (100) nach einem der Ansprüche 1 bis 10, wobei bei dem Abbilden der dreidimensionalen parametrisierten Flächen (2, 10, 11) auf eine oder mehrere zweidimensionale parametrisierte Flächen die lokale Verzerrung der Abbildung in Abhängigkeit von der Wichtigkeit der Bildbereiche des dreidimensionalen Objekts (1) gewählt wird und/oder die unterschiedliche Wichtigkeit der Bildbereiche durch Gewichtungsfaktoren in dem zu optimierenden Energieterm berücksichtigt wird.

12. Verfahren nach einem der Ansprüche 1 bis 11, wobei zusätzlich einer der folgenden Schritte durchgeführt wird:

    a) Annotation von Punkten und/oder Bereichen und/oder Strukturen in der Abbildung,
    b) interaktive Verfeinerung und/oder Verschiebung der Abbildung basierend auf der aktuellen Abbildung,
    c) Erzeugung von Detailansichten.

**14**

**13.** Verfahren (300) zur Darstellung eines Teilbereichs eines zu untersuchenden Objekts (1) mit den folgenden Schritten:

- Durchführen des Verfahrens (100) nach einem der Ansprüche 1 bis 11,
- Festlegen eines darzustellenden Teilbereichs des zu untersuchenden Objekts (1) durch Kennzeichnen in der zweidimensionalen Darstellung,
- Durchführen eines geeigneten Visualisierungsverfahrens in dem darzustellenden Teilbereich.

**14.** Vorrichtung (101) zum Abbilden eines zu untersuchenden dreidimensionalen Objekts (1), aufweisend:

- eine Flächennetz-Ermittlungseinheit (103), die dazu eingerichtet ist, eine dreidimensionale parametrisierte Fläche (2) festzulegen, welche konform zu einer anatomischen Struktur des zu untersuchenden dreidimensionalen Objekts (1) ist, und eine Umgebung der dreidimensionalen parametrisierten Fläche (2) zu parametrisieren,
- eine Reformatierungseinheit (104), welche dazu eingerichtet ist, die Umgebung der dreidimensionalen parametrisierten Fläche (2) in eine aufgefaltete Form, aufweisend einen Schichtstapel mit einer zweidimensionalen parametrisierten Fläche (3, 4), volumetrisch zu deformieren,
- eine Abtasteinheit (105), welche dazu eingerichtet ist, das zu untersuchende dreidimensionale Objekt (1) in aufgefalteter Form durch Abbilden von der parametrisierten Umgebung der dreidimensionalen parametrisierten Fläche (2) zugeordneten Bildpunkten (5) auf die zweidimensionale parametrisierte Fläche (4) des Schichtstapels darzustellen.

**Claims**

**1.** Computer-executed method (100) for mapping a three-dimensional object (1) to be examined, having the following steps:

- defining a three-dimensional parameterised surface (2) which is conformal with an anatomical structure of the three-dimensional object (1) to be examined,
- parameterising an environment of the three-dimensional parameterised surface (2),
- volumetrically deforming the environment of the three-dimensional parameterised surface (2) into an unfolded form, having a slice stack with a two-dimensional parameterised surface (4),
- representing the three-dimensional object (1) to be examined in an unfolded form by mapping image points (5) associated with the parameterised environment of the three-dimensional parameterised surface (2) onto the two-dimensional parameterised surface (4) of the slice stack.

**2.** Method (100) according to claim 1, wherein

- the three-dimensional parameterised surface (2) is parameterised by a three-dimensional surface grid (3) having a large number of individual elements (6),
- the two-dimensional parameterised surface (4) is parameterised by a two-dimensional grid (7) having a large number of individual elements (8),
- two-dimensional representation of the three-dimensional object (1) to be examined by mapping image points (5) associated with the individual elements (6) of the three-dimensional surface grid (3) onto the individual elements (8) of the two-dimensional grid (7).

**3.** Method (100) according to claim 1 or 2, wherein the three-dimensional parameterised surface (2) is an open surface or a closed surface which, before mapping the three-dimensional parameterised surface (2) onto a two-dimensional parameterised surface (4), is converted into an open three-dimensional parameterised surface (2).

**4.** Method (100) according to one of claims 2 or 3, wherein the individual elements (6, 8) comprise triangles and/or rectangles and/or hexagons and/or other polygons and/or the three-dimensional surface grid (3) is a regular grid or an irregular grid.

**5.** Method (100) according to one of claims 1 to 4, wherein the step of mapping the three-dimensional parameterised surface (2) onto the two-dimensional parameterised surface (4) is achieved by optimising an energy term associated with the three-dimensional parameterised surface (2) and a two-dimensional parameterised surface (4) to be optimised.

6.  Method (100) according to one of claims 1 to 5, wherein, in addition to the three-dimensional parameterised surface (2), a plurality of three-dimensional parameterised offset surfaces (10, 11), which are conformal with the anatomical structure of the three-dimensional object (1) to be examined, are generated and are each mapped onto a two-dimensional parameterised surface, so that the three-dimensional object is mapped onto a three-dimensional slice stack comprising two-dimensional surfaces.

7.  Method (100) according to one of claims 1 to 6, wherein the three-dimensional parameterised offset surfaces (10, 11) are defined by determining normal vectors (12) orthogonal to the three-dimensional parameterised surface (2).

8.  Method (100) according to one of claims 1 to 7, wherein the three-dimensional parameterised offset surfaces (10, 11) comprise offset surface grids (13, 14) which are smoothed by applying grid smoothing methods, wherein overlappings of adjacent normal vectors (12) are avoided.

9.  Method (100) according to claim 7, wherein the three-dimensional offset surface grids (13, 14) are explicitly calculated.

10. Method (100) according to one of claims 6 to 9, wherein the rigidity within the slices and the angular distortion between the slices is modeled separately with the energy term.

11. Method (100) according to one of claims 1 to 10, wherein when mapping the three-dimensional parameterised surfaces (2, 10, 11) onto one or more two-dimensional parameterised surface(s), the local distortion of the image is chosen as a function of the importance of the image regions of the three-dimensional object (1) and/or the different importance of the image regions is taken into consideration by way of weighting factors in the energy term to be optimised.

12. Method according to one of claims 1 to 11, wherein, in addition, one of the following steps is carried out:

    a) annotation of points and/or regions and/or structures in the image,
    b) interactive refinement and/or shifting of the image based on the current image,
    c) generation of detailed views.

13. Method (300) for representing a section of an object (1) to be examined having the following steps:

    - carrying out the method (100) according to one of claims 1 to 11,
    - defining a section for representation of the object (1) to be examined by identification in the two-dimensional representation,
    - carrying out a suitable visualisation method in the section for representation.

14. Device (101) for mapping a three-dimensional object (1) to be examined, having:

    - a surface grid-determining unit (103) which is adapted to define a three-dimensional parameterised surface (2) which is conformal with an anatomical structure of the three-dimensional object (1) to be examined, and to parameterise an environment of the three-dimensional parameterised surface (2),
    - a reformatting unit (104) which is adapted to volumetrically deform the environment of the three-dimensional parameterised surface (2) into an unfolded form, having a slice stack with a two-dimensional parameterised surface (3, 4),
    - a sampling unit (105) which is adapted to represent the three-dimensional object (1) to be examined in an unfolded form by mapping image points (5) associated with the parameterised environment of the three-dimensional parameterised surface (2) onto the two-dimensional parameterised surface (4) of the slice stack.

**Revendications**

1.  Procédé (100) effectué par un ordinateur de reproduction d'un objet (1) en trois dimensions à examiner, comprenant les stades :

    - on définit une surface (2) paramétrée en trois dimensions, qui est conforme à une structure anatomique de l'objet (1) en trois dimensions à examiner,
    - on paramétrise un environnement de la surface (2) paramétrée en trois dimensions,

- on déforme volumétriquement l'environnement de la surface (2) paramétrée en trois dimensions en une forme dépliée comportant une pile de couches ayant une surface (4) paramétrée en deux dimensions,
- on représente l'objet (1) en trois dimensions à examiner sous forme dépliée par reproduction des pixels (5) associés à l'environnement paramétré de la surface (2) paramétrée en trois dimensions sur la surface (4) paramétrée en deux dimensions de la pile de couches.

**2.** Procédé (100) suivant la revendication 1, dans lequel

- on paramétrise la surface (2) paramétrée en trois dimensions par un réseau (3) de surface en trois dimensions ayant une pluralité d'éléments (6) individuels,
- on paramétrise la surface (4) paramétrée en deux dimensions par un réseau (7) en deux dimensions ayant une pluralité d'éléments (8) individuels,
- la représentation en deux dimensions de l'objet (1) en trois dimensions à examiner s'effectue par reproduction sur les éléments (8) individuels du réseau (7) en deux dimensions de pixels (5) associés aux éléments (6) individuels du réseau (3) de surface en trois dimensions.

**3.** Procédé (100) suivant la revendication 1 ou 2, dans lequel la surface (2) paramétrée en trois dimensions est une surface ouverte ou une surface fermée, qui, avant la reproduction de la surface (2) paramétrée en trois dimensions, sur une surface (4) paramétrée en deux dimensions, est transformée en une surface (2) ouverte paramétrée en trois dimensions.

**4.** Procédé (100) suivant l'une des revendications 2 ou 3, dans lequel les éléments (6, 8) individuels comprennent des triangles et/ou des quadrilatères et/ou des hexagones et/ou d'autres polygones et/ou le réseau (3) de surface en trois dimensions est un réseau régulier ou un réseau irrégulier.

**5.** Procédé (100) suivant l'une des revendications 1 à 4, dans lequel on réalise le stade de la reproduction de la surface (2) paramétrée en trois dimensions sur la surface (4) paramétrée en deux dimensions, en optimisant un terme d'énergie associé à l'une des surfaces (2) paramétrées en trois dimensions et à l'une des surfaces (4) paramétrées en deux dimensions à optimiser.

**6.** Procédé (100) suivant l'une des revendications 1 à 5, dans lequel, en plus de la surface (2) paramétrée en trois dimensions, on produit une pluralité de surfaces (10, 11) décalées paramétrées en trois dimensions, qui sont conformes à la structure anatomique de l'objet (1) en trois dimensions à examiner et on les reproduit chacune sur une surface paramétrée en deux dimensions, de manière à reproduire l'objet en trois dimensions sur une pile de couches en trois dimensions constituée de surfaces en deux dimensions.

**7.** Procédé (100) suivant l'une des revendications 1 à 6, dans lequel on définit les surfaces 10, 11) de décalage paramétrées en trois dimensions, en déterminant des vecteurs (12) normaux orthogonaux à la surface (2) paramétrée en trois dimensions.

**8.** Procédé (100) suivant l'une des revendications 1 à 7, dans lequel les surfaces (10, 11) de décalage paramétrées en trois dimensions comprennent des réseaux (13, 14) de surface de décalage, qui sont lissés par application de procédés de lissage de réseau quadrillé, en évitant des chevauchements de vecteurs (12) normaux voisins.

**9.** Procédé (100) suivant la revendication 7, dans lequel on calcule explicitement les réseaux (13, 14) de surface de décalage en trois dimensions.

**10.** Procédé (100) suivant l'une des revendications 6 à 9, dans lequel on modélise séparément, par le terme d'énergie, l'équidistance au sein des couches et la déformation angulaire entre les couches.

**11.** Procédé (100) suivant l'une des revendications 1 à 10, dans lequel, lors de la reproduction des surfaces (2, 10, 11) paramétrées en trois dimensions sur une ou sur plusieurs surfaces paramétrées en deux dimensions, on sélectionne la déformation locale de la reproduction en fonction de l'importance des parties d'image de l'objet (1) en trois dimensions et/ou on tient compte de l'importance différente des parties d'image par des facteurs de pondération dans le terme d'énergie à optimiser.

**12.** Procédé suivant l'une des revendications 1 à 11, dans lequel on effectue, en outre, l'un des stades suivants :

a) annotation de points et/ou de régions et/ou de structures dans la reproduction,
b) affinement interactif et/ou déplacement de la reproduction sur la base de la reproduction en cours,
c) production de vues de détail.

**13.** Procédé (300) de représentation d'une région partielle d'un objet (1) à examiner, comprenant les stades suivants :

- on effectue le procédé (100) suivant l'une des revendications 1 à 11,
- on définit une région partielle à représenter de l'objet (1) à examiner par caractérisation dans la représentation en deux dimensions,
- on effectue un procédé de visualisation approprié dans la région partielle à représenter.

**14.** Installation (101) de reproduction d'un objet (1) en trois dimensions à examiner, comportant :

- une unité (103) de détermination de réseau de surface, qui est conçue pour définir une surface (2) paramétrée en trois dimensions, qui est conforme à une structure anatomique de l'objet (1) en trois dimensions à examiner et pour paramétrer un environnement de la surface (2) paramétrée en trois dimensions,
- une unité (104) de reformatage, qui est conçue pour déformer volumétriquement l'environnement de la surface (2) paramétrée en trois dimensions en une forme dépliée comportant une pile de couches ayant une surface (3, 4) paramétrée en deux dimensions,
- une unité (105) d'étalonnage, qui est conçue pour représenter, sur la surface (4) paramétrée en deux dimensions de la pile de couches, l'objet (1) en trois dimensions à examiner sous forme dépliée par reproduction de l'environnement paramétré de pixels (5) associés à la surface (2) paramétrée en trois dimensions.

FIG 1

100

1.I

1.II

1.III

FIG 2

2   6

7

b

1   3

8

4   4

EP 3 155 597 B1

FIG 3

# FIG 4

# FIG 5

# FIG 6

FIG 7

21    25    20

ADR V W    M LT VW

ax    cor    sag

22    23    24

FIG 8

4    3    2    1    up    vw

FIG 9

FIG 10

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2008049991 A1

**In der Beschreibung aufgeführte Nicht-Patentliteratur**

- **SORKINE et al.** As rigid as possible surface modeling. *Symposium on Geometriy processing,* 2007, vol. 4
- A local/global approach to mesh parameterization. **LIU et al.** Computer Graphics Forum. Wiley Online Library, 2008, vol. 27, 14951504
- GPU based ARAP deformation using volumetric lattices. **ZOLLHÖFER et al.** Eurographics 2012Short Papers. The Eurographics Association, 2012, 8588
- **KANITSAR A et al.** Advanced curved planar reformation: flattening of vascular structures. *IEEE VISUALIZATION,* 2003
- **W. KABSCH.** A - solution for the best rotation to relate two sets of vectors. *Acta Crystallographica Section A,* 1976, vol. 32 (5), 922-923
- **U. PINKALL ; K. POLTHIER.** Computing discrete minimal surfaces and their conjugates. *Experimental mathematics,* 1993, vol. 2 (1), 15-36